Europäisches Patentamt

European Patent Office

Office européen des brevets

(19)

(11) Publication number: **0 052 926**
**B1**

(12) **EUROPEAN PATENT SPECIFICATION**

(45) Date of publication of patent specification: **24.04.85**

(51) Int. Cl.⁴: **A 61 L 11/00, A 61 L 2/04**

(21) Application number: **81304607.5**

(22) Date of filing: **05.10.81**

(54) **Device for destroying bacterial flora.**

(30) Priority: **21.11.80 AU 6584/80**

(43) Date of publication of application:
**02.06.82 Bulletin 82/22**

(45) Publication of the grant of the patent:
**24.04.85 Bulletin 85/17**

(84) Designated Contracting States:
**AT BE CH DE FR GB IT LI LU NL SE**

(56) References cited:
**EP-A-0 020 157**
**DE-A-2 503 605**
**US-A-2 708 074**

(73) Proprietor: **Gemmell, Leslie Wallace**
**72 Silverdale Road**
**Eaglemont Victoria (AU)**

(72) Inventor: **Gemmell, Leslie Wallace**
**72 Silverdale Road**
**Eaglemont Victoria (AU)**

(74) Representative: **Barrett, James William et al**
**A.A. THORNTON & CO. Northumberland House**
**303-306 High Holborn**
**London WC1V 7LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to a device for destroying bacterial flora by the sterilization of contaminated waste which contains the bacterial flora.

In hospitals, for example, waste materials are often disposed of by pouring the material down sinks and the like. This waste material may therefore gather in the S-bends or the like, of drainage pipes where bacterial flora in the waste material flourishes.

The bacterial flora which builds up on the drainage pipe is extremely dangerous, particularly in hospitals where it is possible that sterile equipment may be contaminated by the bacterial flora. Indeed, it has been known for a patient in a hospital to contract a sickness which has been attributed to bacterial flora contamination of instruments which has been used in connection with the patient.

The present invention concerns an improvement to the device disclosed in European Patent Application No. 80301799.5 (Publication No. 20157) and invented by the inventor of the present application. In the earlier European patent application (which was published after the priority date of the present application) there is disclosed a device for destroying bacterial flora by sterilization of contaminated waste which contains the bacterial flora, comprising a housing having an inlet to allow waste material to pass into said housing and heating means in said housing for heating the waste material such that any bacterial flora which gathers in or on the housing may be sterilized by heat from said heating means.

An object of the present invention is to provide such a device with means for controlling the heating means to maintain the temperature of the waste material in the housing above a predetermined value, so that the material does not completely cool between sterilizing steps, and also to prevent the waste material from being heated to too high a temperature.

The invention includes a device for destroying bacterial flora by sterilization of contaminated waste which contains the bacterial flora, comprising a housing, said housing having an inlet to allow waste material to pass into said housing, and heating means in said housing for heating the waste material such that any bacterial flora which gathers in or on said housing may be sterilized by heat from said heating means, to thereby destroy said bacterial flora wherein said device further includes means for controlling said heating means to maintain the temperature in said housing above a first predetermined value said means including sensing means for sensing the temperature of water in the housing and energizing the heater means when the temperature drops below the predetermined value, and second sensing means to determine if the temperature in the housing exceeds a second predetermined value and to cause the heating means to be de-energized if the temperature exceeds the second predetermined value.

The invention also includes a device for destroying bacterial flora by sterilization of contaminated waste which contains the bacterial flora, said device comprising a housing, said housing having an inlet to allow waste material to pass into said housing, and heating means in said housing for heating the waste material such that any bacterial flora which gathers in or on said housing may be sterilized by heat from said heating means, wherein said device further comprises a liquid temperature sensor for sensing the temperature of liquid within the housing, a housing temperature sensor for sensing the temperature of the housing, a probe mounted in the housing for sensing the level of liquid within the housing so as to change states if the level falls below a predetermined level, and temperature controlling means electrically connected to the liquid temperature sensor, the housing temperature sensor, the probe and the heating means, said temperature controlling means being arranged to energize the heating means for a predetermined time period if the sensed liquid temperature is below a first predetermined temperature value and if the liquid level is above its predetermined level; provided that, the housing temperature remains less than a second predetermined temperature value and the liquid level remains above its predetermined level, whereby the device safely destroys bacterial flora associated with contaminated waste.

In the above-mentioned earlier European patent application, the device is formed as a waste trap and comprises an elongate housing including means for connection to a drainage pipe, and heating means.

The present invention includes a waste trap device for destroying bacterial flora by sterilization of contaminated liquid waste which contains the bacterial flora, said device comprising an elongate housing including means for connection to a drainage pipe, and heating means wherein the longitudinal axis of the housing is transverse to that of the drainage pipe so as to form at least part of water plug and, thereby facilitate connection and maintenance of the device, the heating means being for heating the waste material such that any bacterial flora which gathers in or on said housing may be sterilized by heat from said heating means, means for controlling the heating means so as to maintain the temperature in the liquid waste above a first predetermined value, said temperature controlling means further comprising means for sensing the temperature in the housing, said temperature sensing means being located in a lower portion of said housing, and a low level probe mounted through an upper portion of the housing so as to project into said housing the probe interfaced with said heating means for sensing when liquid in the housing drops below a predetermined level and for causing the heating means to be de-energized when the liquid level drops below this predetermined level, the means for controlling the heating means responsive to the housing temperature sensing means for de-energizing the heating means when the housing exceeds a second predetermined temperature value, whereby the device safely destroys bacterial flora associated with contaminated waste.

We have been made aware of US Patent Specification No. 2708074 and German Offen-legungsschrift No. 2503605 both of which are concerned with destroying bacterial flora in hospital waste by sterilization. In the devices disclosed in these specifications steam is introduced into a housing to sterilize waste contained therein. In neither specification is there disclosed control means for controlling heating means to maintain the temperature of the waste material in the housing above a predetermined value and to prevent the waste material from being heated to too high a temperature.

In order that the invention may be well understood, an embodiment thereof which is given by way of example only, will now be described, reference being had to the accompanying drawings, in which:

Figure 1 is a cross-sectional view of one embodiment of the invention;

Figure 2 is a circuit diagram for the embodiment of Figure 1.

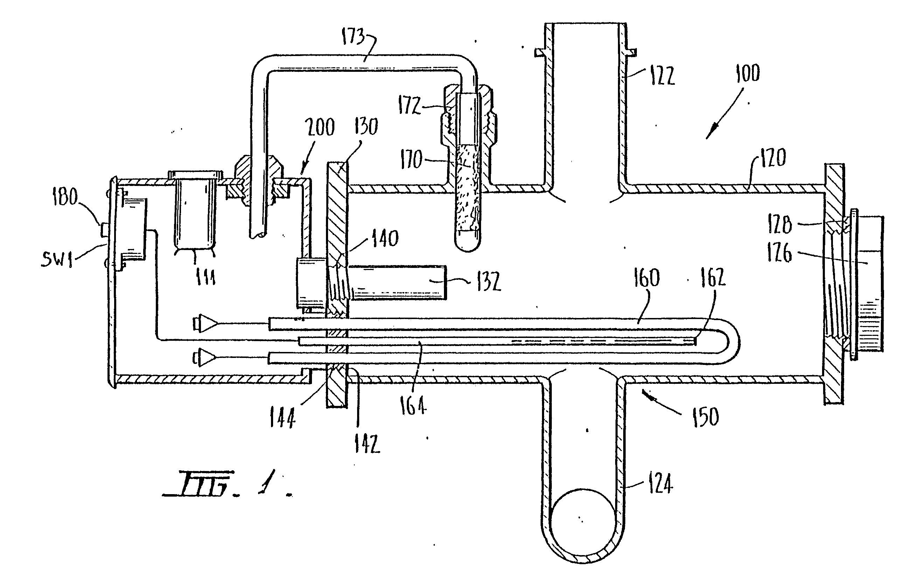

Referring first to Figure 1 the device 100 embodying the invention is shown located in a water plug section 150 of a drainage pipe for example an S-bend. The device 100 includes a housing 120 having an inlet pipe 122 and an outlet pipe 124 both of which form part of the S-bend. The inlet and outlet pipes 122 and 124 are preferably provided with coupling flanges (not shown) and heat resistant gaskets which allow the pipes 122 and 124 to be connected to the remainder of the drainage pipe (not shown).

The housing 120 has one end provided with a removable plug 126 and a heat resistant gasket 128 which allows the interior of the housing to be inspected for purposes of maintenance and the like. The other end is permanently closed by a member 130 which has two openings, the purpose of which will be evident from the following. A first opening 140 receiving a closed sleeve 132 for housing an IC temperature sensor (not shown). The sleeve 132 is screwed into opening 140 by co-operating screw threads on the sleeve and opening. The second opening 142 receives a screw threaded plug 144 which carries a heating element 160 and a temperature sensor probe pocket 162 and the plug 144 is screwed into opening 142 to close that opening.

The pocket 162 carries a sensor probe 164 for measuring the temperature in the interior of the housing 120.

The probe 164 is preferably a bellows type heat sensor preferably made by TL.L. Thermatic Controls Ltd. Canada, No. 53438.

The housing 120 also has a liquid level detecting probe 170 projecting thereinto. The probe 170 is preferably a "low level probe" made and sold by L. W. Gemmell, 72 Silverside Road, Eaglemont Victoria 3084, Australia and the heating element 160 is made and sold by Stokes Australia Ltd., Sullon Street, Brunswick, Victoria, Australia.

The probe 170 is located in a housing 172 provided on the top of the housing 120 and the probe monitors the liquid level in the housing. If the liquid level falls below the level of the probe the operation of the unit will be stopped as will be described hereinbelow. Should the probe 170 fail, the probe 164, as will be described hereinbelow, also cuts off operation of the device and the device will not operate again until manual reset button 180 is depressed, to close manual reset switch SW1.

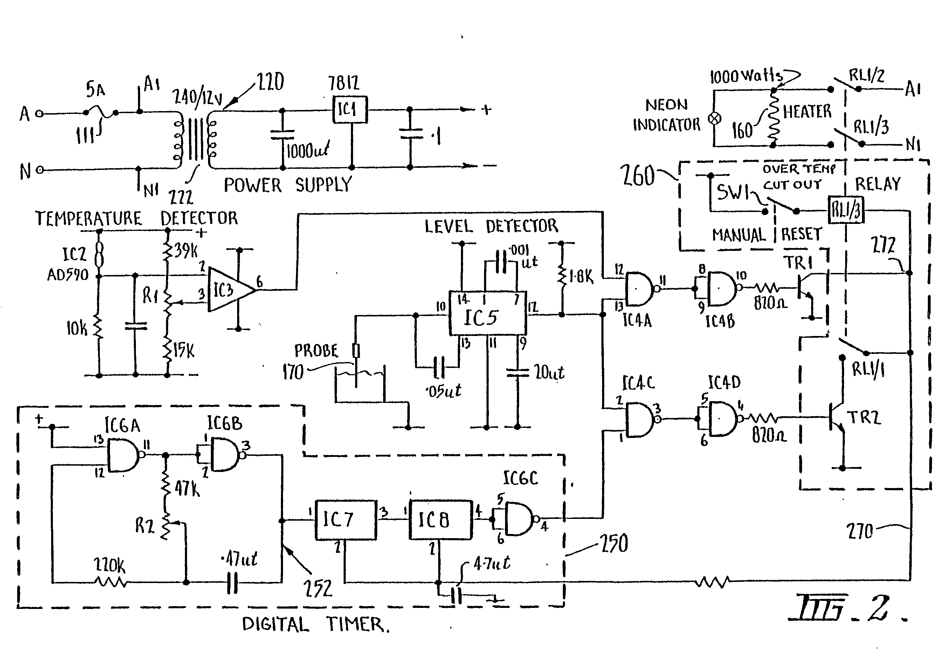

As can be seen from figure 1 a compartment 200 is located at one end of the housing 120 and the IC temperature sensor in sleeve 132, heating element 160 and probe 162 communicate with the compartment. Also the probe 170 communicates with the compartment 200 by cable 173. The compartment 200 housing the circuitry and timer etc. for operating the device which will be described with reference to figure 2.

As shown in figure 2 a conventional 240V power supply is stepped down to 12V by a transformer 222. The stepped down 12V supply is regulated by a voltage regulator, IC1, which is a 12VDC voltage regulator No. 7812 made by Fairchild, and smoothed by filter capacitors as shown. The 12V supply is converted to DC by a rectified diode (not shown) and is used to power the logic circuit to be described thereinafter. A 5 amp fuse 111 is provided in the 240V power supply as shown.

The 240V supply is also connected with heating element 160 through switches RL1/2 and RL1/3 to power the heating element 160. A neon indicator lamp 224 is provided in parallel with the heating element to indicate that power is being supplied to the heating element 160.

The heating element is controlled by the IC temperature sensor IC2 which is located in sleeve 132. The sensor IC2 is preferably a temperature sensor no. AD 590JH made by Analog Devices. The sensor IC2 produces a constant current output proportional to the temperature of the liquid in housing 120, which current is fed to ground via a resistor as shown to develop a voltage proportional to temperature. This voltage is compared with a preset voltage of 3.08V across variable resistor R1 which is equal to a temperature of 35°C. An operation amplifier IC3 (no. 741C made by National Semiconductors) with open loop gain is used to compare the sensor and the voltage across resistor R1 and produce a high output when the temperature is below 35°C. The output of the operational amplifier is applied to one input of a two input NAND gate IC4A. The other input to NAND gate IC4A comes from probe 170 which will be described in detail hereinafter.

The output of gate IC4A is fed to a similar gate IC4B and then through a resistor to a switching transistor TR1. The switching transistor is connected to a relay which closes switches RL1/1, RL1/2 and RL1/3 when power is supplied to the relay. Accordingly if the sensor IC2 senses a temperature below 35°C the output of the operational amplifier IC3 is high. The output from probe 170 is high if the water level in housing 120 is in the safe level condition i.e. above a certain level so that gate IC4A is supplied with two high inputs and therefore supplies a low input to gate IC4B which applies a high signal to transistor TR1 to turn the transistor on. This in turn powers relay RL1/3 to close switches RL1/1 to RL1/3 and power is supplied to heating element 160 to heat the water and waste material in housing 120. The switch RL1/1 ensures that relay RL1/3 remains latched to continue supply power to the heating element.

The probe 170 ensures that heating element 160 is not powered if the water level in housing 120 is below a predetermined level i.e. below the probe 170, wherein the heating element may burn out or be otherwise damaged due to lack of water in the housing 120.

If the liquid level is above a predetermined level i.e. above element 160, the output from the probe 170 and Fluid Detector IC5, which is preferably a Fluid Detector LM 1830 made by National Semiconductors is high thereby operating gate IC4A as described above. If the liquid level is lower than the predetermined level established by probe 170 the output from sensor IC5 is low and accordingly the output from gate IC4A is high the output from gate IC4B is low and transistor TR1 is not switched on. Therefore relay RL1/3 does not close switches RL1/1 to RL1/3 and power is not supplied to heating element 160. Power will not be supplied to heating element 160 until the liquid level is above the probe 170 and the temperature sensor IC2 senses a temperature below 35°C.

The probe 170 and temperature sensor IC5 will also stop supplying power to the heating element 160 if during a heating cycle the liquid drops below probe 170, which may occur if many heating cycles have occurred and no water has passed into the housing 120. The output of sensor IC5 is applied to an input of a two input NAND gate IC4C. The other input to IC4C is from digital timer 250 which will be described hereinafter. During a heating cycle input to gate IC4C from timer 250 will be high. Accordingly gate IC4C will apply a low output to gate IC4D which will apply a high signal to transistor TR2 to turn the transistor on. It will therefore be seen that relay RL1/3 is powered by the circuit label 260.

If the liquid level in housing 120 drops below the level of probe 170 the output from sensor IC5 will go low therefore causing the gate IC4C to go high and the output of gate IC4D to go low thereby turning off transistor TR2 and disrupting power supply to relay RL1/3 to open switches RL1/1 to RL1/3 to cut off power to heating element 160.

The NAND gates IC4A to C are preferably made by National Semiconductor under no. 4011C.

The time the heating element 160 is powered to heat the liquid in the housing 120 is desirably about 17 minutes. This time is controlled by digital timer 250 which comprises an oscillator 250 which includes NAND gates IC6A and IC6B and which is continually in operation, counter IC7 and IC8 and NAND IC6C. The period of oscillation of the oscillator 252 is set by variable resistor R2 and applies a pulse to counter IC7 once every two seconds.

The counter IC7 is a seven stage counter and supplies a pulse from pin 3 to IC8 for every 128 pulses received from the oscillator 252. The counter IC8 is the same as the counter IC7 but applies a pulse from its pin 4 for every 32 pulses received. The output from counter IC8 is applied to gate IC6C which applies a lower output to NAND gate IC4C after every 4,096 oscillations of oscillator 252. Accordingly a high output is applied to gate IC4D and a low output is applied to transistor TR2 to turn the transistor off thereby disrupting power supply to relay RL1/3 to cut off power supply to the heating element 160.

The counters IC7 and IC8 are reset and commence counting on receiving of signals on line 270. When no power is supplied to relay RL1/3 due to transistor TR2 being in the off state point 272 experiences a high voltage which when applied to pin 2 of the counters IC7 and IC8 prevents the counters from counting. When the relay is powered to in turn close switches RL1/1 to RL1/3 the point 272 experiences low voltage which is applied to pin 2 of counters IC7 and IC8 and this results in the counters resetting to zero and allows the counters to commence counting, until the counter IC8 applies its high signal gate IC6C which as noted above cuts off the power to heating element 160 to end a heating cycle. The counters IC7 and IC8 are seven stage counters no. 4024 made by Texas Instruments and the gates IC6A and IC6C are Quad 2 input NAND gates no. 4011C made by National Semiconductors.

Further still a over temperature cut out switch SW1 is provided which is connected to bellows type probe 184. If the temperature in housing 120 reaches 120°C the probe 164 expands and opens the switch SW1 to deactivate relay RL1/3 and cut off power to the heating element 160. The switch SW1 must be manually closed to allow the relay to be reactivated. Accordingly if for some reason the probe 170 etc should fail and not cut off power to the heating element 160 the probe 164 will sense that the heating element 160 is over heating and disrupt power to the heating element.

Accordingly the device will automatically heat the contents of the housing 120 for a period of 17 minutes and will automatically shut off power to the heating element after that time has elapsed and again activate the heating element if the temperature in the housing drops below 35°C. Also if the liquid level drops below a certain level or the temperature in the housing rises above a certain level power to the heating element will be disrupted thereby prevent damage to the heating element 160 or to the device itself.

When actuated the heating element 160 which is in contact with water and waste material in the S bend of a drainage pipe in which the device accordingly to the present embodiment is located, boils the water and waste material which forms the water plug in the S bend and which is located in the housing 160 to destroy bacteria therein. The boiling action is the cylinder causes boiling water to bubble through the S bend 150 and heat from the boiling water is circulated throughout the inlet and outlet pipe to the S bend to destroy any bacteria which may adhere to the surfaces of these members. Any bacteria which of course passes out of the S bend is not a problem since it is not capable of contaminating sterile equipment.

Heat resistant washers (not shown) may also be provided for coupling the device 100 in a pipe to prevent heat conductions beyond the heat resistant washers. Steam trap (not shown) may also be provided at the top of the pipe between a sink or the like and the device 100 to prevent steam from escaping from the pipe.

It should also be noted that the integrated circuits shown in figure 2 will have power supplied to them from the 12V supply and have their other pins connected in accordance with the details shown in figure 2 and the manufacturers specification.

Since modifications within the scope of the invention may readily be effected by persons skilled within the art, it is to be understood that this application is not limited to the particular embodiment described by way of example hereinabove.

**Claims**

1. A device for destroying bacterial flora by sterilization of contaminated waste which contains the bacterial flora, comprising a housing (120), said housing having an inlet (122) to allow waste material to pass into said housing (120), and heating means (160) in said housing for heating the waste material such that any bacterial flora which gathers in or on said housing may be sterilized by heat from said heating means, to thereby destroy said bacterial flora wherein said device further includes means (130, IC2, IC3, IC4, A, B) for controlling said heating means to maintain the temperature in said housing above a first predetermined value said means including sensing means for sensing (IC2) the temperature of water in the housing and energizing the heater means when the temperature drops below the predetermined value, and second sensing means (164) to determine if the temperature in the housing exceeds a second predetermined value and to cause the heating means to be de-energized if the temperature exceeds the second predetermined value.

2. A device according to claim 1, wherein the heating means comprises a heating element (160) and the housing comprises a cylinder (120) located in the water plug of a drainage pipe, the heating element (160) extending longitudinally in the cylinder (120) such that waste material flushed down the sink with water gathers in the cylinder whereby the water is boiled by the heating element (160) and heat is conducted through the drainage pipe via steam and the boiling action of the water and waste material to destroy bacteria in the water as well as bacteria which collects on the inside of the drainage pipe.

3. A device according to claim 1 or 2, wherein the first predetermined temperature value is 35°C and the second predetermined temperature value is 120°C.

4. A device according to any one of the preceding claims wherein said device includes a low level probe (170) for sensing when liquid in said housing drops below a predetermined level and for causing the heating means (160) to be de-energized when the liquid level drops below the predetermined level.

5. A device according to any one of the preceding claims, wherein said second sensing means (164) includes a bellows (164).

6. A device according to claim 4, where an electric control means (220, 250, 260) is provided to control the operation of said heating means (160), said control means being responsive to the output of the temperature sensing means (IC2) and said low level probe (170), said control means including a relay (RL1/3) which is actuated when the temperature sensed by the temperature sensing means (IC/2) is below the predetermined value and when the water level sensed by the low level probe (170) is above the predetermined level, said relay, when actuated, closing switching means (RL1/1 ... 3) for allowing the heating means (160) to be energized.

7. A device according to claim 7 where the outputs from the low level probe (170) and the temperature sensing means (IC2) are applied to gate means (IC4A, IC4B) which turns on a switching transistor (TR1) when the liquid level is above the predetermined level and the temperature is below the predetermined temperature, said switching transistor (TR1), when switched on, causes the relay (RL1/3) to be energized to close said switching means (RL1/1 ... 3).

8. A device according to any one of the preceding claims wherein timer means (250) is provided to de-energize the heating means (160) after the heating means (160) has been energized for a predetermined time interval.

9. A device according to claim 8 when appended to claim 7 or 8, wherein said timer means (250) comprises counter means (IC7, IC8) which are provided with a signal at the commencement of operation of the heating means to set the counter means (IC7, IC8) counting, said counter means applying an output to second gate means (IC6C, IC4C, IC4D), after a predetermined number of counts indicated of the predetermined time interval, to turn a second switching transistor (TR2) off to de-activate said relay (RL1/3) to open the switching means (RL1/1 ... 3) to de-energize said heating means (160).

10. A device according to claim 5 wherein said over temperature sensor (164) includes a bellows which expands to open a switch (SW1) to de-energize said heating means (160) when the temperature exceeds the predetermined value.

11. A device for destroying bacterial flora by sterilization of contaminated waste which contains the bacterial flora, said device comprising a housing (120), said housing having an inlet (122) to allow waste material to pass into said housing, and heating means (160) in said housing (120) for heating the waste material such that any bacterial flora which gathers in or on said housing may be sterilized by heat from said heating means (160), wherein said device further comprises a liquid temperature sensor (IC2) for sensing the temperature of liquid within the housing (120), a housing temperature sensor (164) for sensing the temperature of the housing, a probe (170) mounted in the housing for sensing the level of liquid within the housing (120) so as to change states if the level falls below a predetermined level, and temperature controlling means (250, 260, 130, IC2—4) electrically connected to the liquid temperature sensor (IC2), the housing temperature sensor, the probe and the heating means, said temperature controlling means (250, 260) being arranged to energize the heating means (160) for a predetermined time period if the sensed liquid temperature is below a first predetermined temperature value and if the liquid level is above its predetermined level; provided that, the housing temperature remains less than a second predetermined temperature value and the liquid level remains above its predetermined level; whereby the device safely destroys bacterial flora associated with the contaminated waste.

12. A device according to claim 11, wherein the housing (120) includes means (126) for inspecting the interior of the housing and further wherein the heating means (160) comprises a heating element and the housing comprises a cylinder located in a water plug of the drainable pipe, the heating element (160) extending longitudinally in the cylinder such that waste material flushed down the sink with water gathers in the cylinder whereby the water is boiled by the heating element and heat is conducted through the drainable pipe via steam and the boiling action of the water and waste material to destroy bacteria in the water as well as bacteria which collects on the inside of the drainage pipe.

13. A device according to claim 11 or 12 wherein the temperature controlling means (250, 260 IC2—4, 132) includes a relay (RL1) which is actuated when the temperature sensed by the liquid temperature sensor is below the first predetermined temperature value and when the liquid level sensed by the probe (170) is above a predetermined level, said relay (RL1), when actuated allowing the heating means to be energized.

14. A device according to claim 13 where the outputs from the probe (170) and the liquid temperature sensor (IC2) are applied to gate means (IC4) which turns on a switching transistor (TR1) when the liquid level is above the predetermined level and the temperature is below the first predetermined temperature value, said switching transistor, when switched on, causes the relay to be energized and thereby activates the heating means.

15. A device according to claim 11, 12, 13 or 14, further comprising a timer means (250) to de-energize the heating means (160) after the heating means (160) has been energized for the predetermined time period.

16. A device according to claim 15, wherein said timer means comprises counter means (IC7, IC8) which are provided with a signal of the commencement of operation of the heating means (160) to set the counter means counting; a second gate means (IC6), the said counter means (IC7, IC8) applying an output to the second gate (IC6) means after a predetermined number of counts indicative of the predetermined time interval; a switching transistor (TR2) connected to the second gate means and a relay (RL1/1) controlled by the switching transistor (TR2) so that upon an output generated by the counter means the switching transistor de-activates the relay so as to de-energize said heating means.

17. A device according to any one of claims 11 to 16, wherein the housing temperature sensor (164) includes a bellows and a switch (SW1) such that when the bellows expands, it opens the switch to de-energize said heating means (160) when the temperature exceeds the second predetermined temperature value.

18. A device according to any one of claims 11 to 16, wherein said device includes a manual reset switch (SW1) interconnected to the housing temperature sensor (164) for preventing the heating means (160) from being re-activated after it has been de-energized as a result of the housing temperature exceeding the second predetermined value until said switch (SW1) is manually reset.

19. A waste trap device for destroying bacterial flora by sterilization of contaminated liquid waste which contains the bacterial flora, said device comprising an elongate housing (120) including means (122, 124) for connection to a drainage pipe, and heating means (160) wherein the longitudinal axis of the housing (120) is transverse to that of the drainage pipe so as to form at least part of water plug and, thereby facilitate connection and maintenance of the device, the heating means (160) being for heating the waste material such that any bacterial flora which gathers in or on said housing may be sterilized by heat from said heating means, means for controlling (250, 260, IC2—4, 132) the heating means (160) so as to maintain the temperature in the liquid waste above a first predetermined value, said temperature controlling means further comprising means (164) for sensing the temperature in the housing, said temperature sensing means (164) being located in a lower portion of said housing, and a low level probe (170) mounted through an upper portion of the housing so as to project into said housing (120) the probe (170) interfaced with said heating means (160) for sensing when liquid in the housing drops below a predetermined level and for causing the heating means (160) to be de-energized when the liquid level drops below this predetermined level, the means for controlling (250, 260, IC2—4, 132) the heating means responsive to the housing temperature sensing means (164) for de-energizing the heating means (160) when the housing exceeds a second predetermined temperature value, whereby the device safely destroys bacterial flora associated with contaminated waste.

20. A waste trap device as defined in claim 19 further comprising a manually operable switch (SW1) interconnected with the housing temperature sensing means (164) and the means for controlling (250, 260, IC2—4, 132) the heating means so as to require the manual resetting of the switch (SW1) if the housing temperature exceeds the second predetermined temperature value.

21. A waste trap device as defined in claims 19 or 20 wherein the housing further comprises an inspection opening at one of its ends so as to facilitate maintenance and inspection of the device.

22. A waste trap device as defined in claim 20 wherein the elongate housing (126) comprises a cylinder (120) for location in a water plug of the drainage pipe wherein the heating means (160) comprises a heating element (160) extending longitudinally in the cylinder such that liquid waste flushed down a sink associated with the drainage pipe gathers in the cylinder (120) and is boiled by the heating element with the heat conducted through the drainage pipe via steam and the boiling action of the liquid waste so as to destroy bacteria in the liquid waste as well as bacteria which collects on the inside of the drainage pipe.

23. A waste trap device as defined in claim 22 wherein the means for controlling the heating means (250, 260, IC2) includes a liquid temperature sensor (IC2) for sensing the temperature of the liquid within the housing.

24. A waste trap device as defined in claim 23 wherein the means for controlling (250, 260, IC2—4, 132) the heating means includes a relay (RL1) which is actuated when the temperature sensed by the liquid temperature sensor (IC2) is below a first predetermined value and when the liquid level sensed by the low level probe (170) is above the predetermined level; said relay (RL1), when actuated allowing the heating means (160) to be energized.

25. A waste trap device as defined in claim 24 wherein the means for controlling (250, 260, IC2—4, 132) the heating means (160) includes gate means (IC4) and a switching transistor (TR1), the gate means (IC4) interconnected with the outputs from the low level probe (170) and the liquid temperature sensor (IC2) so as to turn on the switching transistor (TR1) when the liquid level is above the predetermined level and the temperature is below the first predetermined temperature value, the switching transistor (TR1) when switched on causing the relay (RL1) to be

energized thereby activating the heating means.

26. A waste trap device as defined in any one of claims 19 to 25, further comprising timer means (250) to de-energize the heating means (160) after the heating means (160) has been energized for a predetermined time period.

27. A waste trap device as defined in claim 26 wherein the timing means (250) comprises counter means (IC7, IC8) that are provided with a signal at the commencement of operation of the heating means to set the counter means (IC7, IC8) counting, a second gate means (IC6) the counter means applying an output to the second gate means (IC6) after a predetermined number of counts indicative of the predetermined time period; a second switching transistor (TR2) connected to the second gate means (IC6) and a relay (RL1/1) controlled by the second switching transistor (TR2) so that upon an output generated by the counting means (IC7, IC8) the second switching transistor de-activates the relay (RL1/1) so as to de-energize the heating means (160).

28. A waste trap device as defined in any one of claims 19 to 27, wherein the housing temperature sensor (164) includes a bellows and a switch (SW1) such that when the bellows expands, it opens the switch (SW1) to de-energize the heating means (160) when the temperature exceeds the second predetermined value.

### ntentansprüche

1. Einrichtung zum Vernichten der bakteriellen Flora durch Sterilisieren des verunreinigten Abfalls, der die bakterielle Flora enthält, bestehend aus einem Gehäuse (120), mit einem Einlaß (122) für die Zuführung des Abfallmaterials in das Gehäuse (120), einer Heizvorrichtung (160) im Gehäuse zum Erhitzen des Abfallmaterials derart, daß jegliche bakterielle Flora, die sich in oder an dem Gehäuse sammelt, durch Hitze von der Heizvorrichtung sterilisiert wird, um dadurch die genannte bakterielle Flora zu zerstören, wobei die Einrichtung weiterhin Vorrichtungen (130, IC2, IC3, IC4 A, B) zum Beeinflussen der Heizvorrichtung aufweist, um die Temperatur in dem Gehäuse oberhalb eines ersten vorbestimmten Wertes zu halten, wobei diese Vorrichtungen einen Sensor (IC2) zum Ermitteln der Temperatur von Wasser und für die Energieversorgung der Heizvorrichtung, wenn die Temperatur unter den vorbestimmten Wert fällt, und einen zweiten Sensor (164) aufweisen, um zu bestimmen, ob die Temperatur in dem Gehäuse einen zweiten vorbestimmten Wert übersteigt und um zu bewirken, daß die Heizvorrichtung abgeschaltet wird, wenn die Temperatur den zweiten vorbestimmten Wert übersteigt.

2. Einrichtung nach Anspruch 1, bei der die Heizvorrichtung ein Heizelement (160) enthält und das Gehäuse einen Zylinder (120) aufweist, der in dem Wasserhahn eines Ablaßrohres angeordnet ist, wobei das Heizelement (160) sich in Längsrichtung im Zylinder (120) erstreckt derart, daß das mit Wasser nach unten in den Ausguß ge-

spülte Abfallmaterial sich in dem Zylinder sammelt, wodurch das Wasser durch das Heizelement (160) zum Kochen gebracht wird und Wärme durch das Ablaufrohr durch den Dampf und das Kochen des Wassers und des Abfallmaterials geleitet wird, um Bakterien im Wasser als auch Bakterien, die sich an der Innenseite des Ablaufrohrs sammeln, zu zerstören.

3. Einrichtung nach Anspruch 1 oder 2, dadurch gekennzeichnet, daß der erste vorbestimmte Temperaturwert 35°C und der zweite vorbestimmte Temperaturwert 120°C betragen.

4. Einrichtung nach einem der vorhergehenden Ansprüche, gekennzeichnet durch eine Niederpegelsonde (170), um das Abfallen der Flüssigkeit im Gehäuse unter einen vorbestimmten Pegel festzustellen und um zu bewirken, daß die Heizvorrichtung (160) abgeschaltet wird, wenn der Flüssigkeitspegel unter den vorbestimmten Pegel fällt.

5. Einrichtung nach einem der vorhergehenden Ansprüche, bei der die zweite Sensoreinrichtung eine Balg (164) enthält.

6. Einrichtung nach Anspruch 4, enthaltend eine elektrische Steuereinrichtung (220, 250, 260) zum Steuern des Betriebs der Heizvorrichtung (160), die auf den Ausgang des Temperaturfühlers (IC2) und der Niederpegelsonde (170) anspricht und ein Relais (RL1/3) enthält, das betätigt wird, wenn die von dem Temperaturfühler (IC2) ermittelte Temperatur unterhalb des vorbestimmten Wertes liegt und wenn der Wasserpegel, der von der Niederpegelsonde (170) ermittelt, wird, oberhalb des vorbestimmten Pegels liegt, wobei das Relais bei Betätigung einen Schalter (RL1/1 ... 3) schließt, der die Energiezufuhr zur Heizvorrichtung (160) erlaubt.

7. Einrichtung nach Anspruch 6, bei der die Ausgänge der Niederpegelsonde (170) und des Temperaturfühlers (IC2) Torschaltungen (IC4A, IC4B) zugeführt werden, die einen Schalttransistor (TR1) einschalten, wenn der Flüssigkeitspegel oberhalb des vorbestimmten Pegels liegt und die Temperatur unterhalb der vorbestimmten Temperatur liegt, und welcher Schalttransistor (TR1) im Einschaltzustand die Erregung des Relais (RL1/3) bewirkt, um die Schaltermittel (RL1/1 ... 3) zu schließen.

8. Einrichtung nach einem der vorhergehenden Ansprüche, bei der ein Zeitgeber (250) vorgesehen ist, um die Heizvorrichtung (160) abzuschalten, nachdem diese für ein vorbestimmtes Zeitintervall mit Energie versorgt worden ist.

9. Einrichtung nach Anspruch 8, oder nach Anspruch 7 und 8, bei der der Zeitgeber (250) einen Zähler (IC7, IC8) enthält, dem ein Signal zu Beginn des Betriebs der Heizvorrichtung zugeführt ist, um den Zähler (IC7, IC8) in den Zählzustand zu versetzen, wobei der Zähler ein Ausgangssignal zweiten Torschaltungen (IC6C, IC4C, IC4D) nach einer vorbestimmten Zahl von Zählungen zuführt, die von dem vorbestimmten Zeitintervall angegeben ist, um einen zweiten Schalttransistor (TR2) auszuschalten, um das Relais (RL1/3) abzuschalten und die Schaltermittel (RL1/1 ... 3) zu öffnen, um die Heizvorrichtung (160) abzuschalten.

10. Einrichtung nach Anspruch 5, bei der der Übertemperaturfühler (164) einen Balgen enthält, der sich ausdehnt, um einen Schalter (SW1) zu öffnen, um die Heizvorrichtung (160) abzuschalten, wenn die Temperatur den vorbestimmten Temperaturwert übersteigt.

11. Einrichtung zum Zerstören bakterieller Flora durch Sterilisieren verunreinigten Abfalls, der die bakterielle Flora aufweist, enthaltend ein Gehäuse (120) mit einem Einlaß (122) zur Zuführung von Abfallmaterial in das Gehäuse, eine Heizvorrichtung (160) im Gehäuse (120) zum Aufheizen des Abfallmaterials derart, daß jegliche bakterielle Flora, die sich in oder an dem Gehäuse sammelt, durch Hitze von der Heizvorrichtung (160) sterilisiert werden kann, wobei die Einrichtung weiterhin einen Flüssigkeitstemperatursensor (IC2) zum Ermitteln der Temperatur der in dem Gehäuse (120) enthaltenen Flüssigkeit, einen Gehäusetemperatursensor (164) zum Ermitteln der Temperatur des Gehäuses, eine im Gehäuse montierte Sonde (170) zum Ermitteln des Flüssigkeitspegels innerhalb des Gehäuses (120), um Zustände zu ändern, wenn der Pegel unter einen vorbestimmten Pegel fällt, und Temperatursteuerungseinrichtungen (250, 260, 130, IC2—4), die elektrisch mit dem Flüssigkeitstemperatursensor (IC2), dem Gehäusetemperatursensor, der Sonde und der Heizvorrichtung verbunden und so eingerichtet sind, daß die Heizvorrichtung (160) für eine vorbestimmte Zeitdauer mit Energie versorgt wird, wenn die ermittelte Flüssigkeitstemperatur unterhalb eines ersten vorbestimmten Temperaturwertes liegt, und wenn der Flüssigkeitspegel oberhalb eines vorbestimmten Pegels liegt, vorausgesetzt, die Gehäusetemperatur bliebt unterhalb eines zweiten vorbestimmten Temperaturwertes und der Flüssigkeitsspiegel bleibt oberhalb seines vorbestimmten Wertes, wobei die Vorrichtung mit Sicherheit die bakterielle Flora zerstört, die in dem verunreinigten Abfall enthalten ist.

12. Einrichtung nach Anspruch 11, bei der das Gehäuse (120) eine Einrichtung (126) zum Inspizieren des Gehäuseinneren aufweist und bei der die Heizvorrichtung (160) ein Heizelement enthält und das Gehäuse aus einem Zylinder besteht, der in einem Wasserhahn des Ablaufrohres angeordnet ist, wobei das Heizelement (160) sich in Längsrichtung im Zylinder derart erstreckt, daß Abfallmaterial, das mit Wasser in den Ablauf hinabgespült worden ist, sich im Zylinder sammelt, wodurch das Wasser von dem Heizelement zum Kochen gebracht wird und Wärme durch das Ablaufrohr durch den Dampf und das Kochen des Wassers und des Abfallmaterials geleitet wird, um Bakterien im Wasser als auch Bakterien, die sich an der Innenseite des Ablaufrohrs sammeln, zu zerstören.

13. Einrichtung nach Anspruch 11 oder 12, bei der die Temperaturregeleinrichtung (250, 260, IC2—4, 132) ein Relais (RL1) enthält, das betätigt wird, wenn die von dem Flüssigkeitstemperaturfühler ermittelte Temperatur unterhalb des ersten vorbestimmten Temperaturwerts liegt und wenn der von der Sonde (170) ermittelte Flüssigkeitspegel oberhalb des vorbestimmten Pegels liegt, wobei das Relais (RL1) bei Betätigung die Energieversorgung der Heizvorrichtung zuläßt.

14. Einrichtung nach Anspruch 13, bei der die Ausgänge der Sonde (170) und des Flüssigkeitstemperaturfühlers (IC2) einer Torschaltung (IC4), zugeführt sind, die einen Schalttransistor (TR1) einschaltet, wenn der Flüssigkeitspegel oberhalb des vorbestimmten Pegels ist und die Temperatur unterhalb des ersten vorbestimmten Temperaturwertes liegt, wobei der Schalttransistor im Einschaltzustand die Erregung des Relais und dadurch die Einschaltung der Heizvorrichtung bewirkt.

15. Einrichtung nach Anspruch 11, 12, 13 oder 14, weiterhin einen Zeitgeber (250) enthaltend, um die Heizvorrichtung (160) abzuschalten, nachdem diese für die vorbestimmte Zeitdauer mit Energie versorgt worden ist.

16. Einrichtung nach Anspruch 15, wobei der der Zeitgeber eine Zähler (IC7, IC8) aufweist, dem ein Signal über den Betriebsbeginn der Heizvorrichtung (160) zugeführt ist, um den Zähler in den Zählzustand zu versetzen; eine zweite Torschaltung (IC6) vorgesehen ist, der von dem Zähler (IC7, IC8) nach einer vorbestimmten Zahl von Zählungen, die dem vorbestimmten Zeitintervall entsprechen, ein Ausgangssignal zugeführt ist; ein Schalttransistor (TR2) mit der zweiten Torschaltung verbunden ist und ein Relais (RL1/1) von dem Schalttransistor (TR2) so gesteuert wird, daß bei Erzeugung eines Ausgangs durch den Zähler der Schalttransistor das Relais abschaltet, um die Heizvorrichtung abzuschalten.

17. Einrichtung nach einem der Ansprüche 11 bis 16, bei der der Gehäusetemperaturfühler (164) einen Balgen und einen Schalter (SW1) enthält derart, daß, wenn der Balgen sich ausdehnt, er den Schalter öffnet, um die Heizvorrichtung (160) abzuschalten, wenn die Temperatur den zweiten vorbestimmten Temperaturwert übersteigt.

18. Einrichtung nach einem der Ansprüche 11 bis 16, enthaltend einen manuellen Rücksetzschalter (SW1), der mit dem Gehäusetemperaturfühler (164) verbunden ist, um zu verhindern, daß die Heizvorrichtung (160) wieder in Betrieb gesetzt wird, nachdem sie als Folge eines Gehäusetemperaturanstiegs über den zweiten vorbestimmten Wert hinaus abgeschaltet worden ist, bis der genannte Schalter (SW1) manuell rückgestellt wird.

19. Abfallauffangvorrichtung zum Zerstören bakterieller Flora durch Sterilisierung von verunreinigtem Flüssigabfall, der die bakterielle Flora enthält, enthaltend ein langgestrecktes Gehäuse (120) mit Einrichtungen (122, 124) zur Verbindung mit einem Ablaufrohr und einer Heizvorrichtung (160), wobei die Längsachse des Gehäuses (120) quer zu der des Ablaufrohres liegt, um wenigstens Teil eines Wasserhahnes zu bilden und um dadurch die Verbindung und die Wartung der Vorrichtung zu erleichtern, wobei die Heizvorrichtung (160) zur Aufheizung des Abfallmaterials vorgesehen ist derart, daß jegliche bakterielle Flora, die sich in oder an dem Gehäuse sammelt, durch die Hitze von der Heizvorrichtung sterilisiert wird, Einrichtungen (250, 260, IC2—4, 132) zum Beeinflussen der Heizvorrichtung (160), um die Temperatur in dem Flüssigabfall oberhalb eines ersten vorbestimmten Wertes zu halten, wobei die Temperaturbeeinflussungseinrichtung weiterhin einen Gehäusetemperaturfühler (164) enthält, der in einem unteren Bereich des Gehäuses angeordnet ist, und eine Niedrigpegelsonde (170) aufweist, die in einem oberen Bereich des Gehäuses so montiert ist, daß sie in das Gehäuse (120) vorsteht, wobei die Sonde (170) zwischen der Heizvorrichtung (160) liegt, um zu ermitteln, wenn die Flüssigkeit im Gehäuse unter einen vorbestimmten Pegel fällt und um die Abschaltung der Heizvorrichtung (160) zu bewirken, wenn der Flüssigkeitspegel unter diesen vorbestimmten Pegel fällt, wobei die Einrichtung (250, 260, IC2—4, 132) zum Beeinflussen der Heizvorrichtung auf den Gehäusetemperatursensor (164) anspricht, um die Heizvorrichtung (160) abzuschalten, wenn das Gehäuse eine zweiten vorbestimmten Temperaturwert übersteigt, wodurch die Vorrichtung mit Sicherheit bakterielle Flora zerstört, die sich in dem verunreinigten Abfall befindet.

20. Abfallauffangvorrichtung nach Anspruch 19, weiterhin enthaltend einen manuell betätigbaren Schalter (SW1), der mit dem Gehäusetemperaturfühler (164) und der Einrichtung (250, 260, IC2—4, 132) zur Beeinflussung der Heizvorrichtung derart verbunden ist, daß ein manuelles Rückstellen des Schalters (SW1) erforderlich ist, wenn die Gehäusetemperatur den zweiten vorbestimmten Temperaturwert übersteigt.

21. Abfallauffangvorrichtung nach den Ansprüchen 19 oder 20, bei der das Gehäuse weiterhin eine Inspektionsöffnung an einem seiner Enden aufweist, um die Wartung und die Inspektion der Vorrichtung zu erleichtern.

22. Abfallauffangvorrichtung nach Anspruch 20, bei der das langgestreckte Gehäuse (126) einen Zylinder (120) zur Anbringung in einem Wasserhahn eines Ablaufrohres enthält, wobei die Heizvorrichtung (160) ein Heizelement (160) enthält, das sich in Längsrichtung im Zylinder derart erstreckt, daß Flüssigabfall, der nach unten in einen mit dem Ablaufrohr verbundenen Ablauf gespült wird, sich in dem Zylinder (120) sammelt und von dem Heizelement zum Kochen gebracht wird, wobei die Hitze mit dem Dampf und durch das Kochen des Flüssigabfalls durch das Ablaufrohr geleitet wird, um Bakterien im Flüssigabfall als auch Bakterien, die sich an der Innenseite des Ablaufrohres gesammelt haben, zu zerstören.

23. Abfallauffangvorrichtung nach Anspruch 22, bei der die Einrichtung (250, 260, IC2) zum Beeinflussen der Heizvorrichtung eine Flüssigkeitstemperaturfühler (IC2) zum Ermitteln der Temperatur der Flüssigkeit im Gehäuse aufweist.

24. Abfallauffangvorrichtung nach Anspruch 23, bei der die Einrichtung (250, 260, IC2—4, 132) zum Beeinflussen der Heizvorrichtung ein Relais (RL1) enthält, das betätigt wird, wenn die von dem Flüssigkeitstemperaturfühler (IC2) ermittelte Temperatur unterhalb einem ersten vorbestimmten Wert liegt und wenn der Flüssigkeitspegel, der von der Niedrigpegelsonde (170) emittelt wird, oberhalb des vorbestimmten Pegels liegt, wobei das Relais (RL1) bei Betätigung die Energiezufuhr zur Heizvorrichtung (160) erlaubt.

25. Abfallauffangvorrichtung nach Anspruch 24, bei der die Einrichtung (250, 260, IC2—4, 132) zum Beeinflussen der Heizvorrichtung (160) eine Torschaltung (IC4) und einen Schalttransistor (TR1) aufweist, wobei die Torschaltung (IC4) mit den Ausgängen der Niedrigpegelsonde (170) und dem Flüssigkeitstemperaturfühler (IC2) verbunden ist, um den Schalttransistor (TR1) einzuschalten, wenn der Flüssigkeitspegel oberhalb des vorbestimmten Pegels und die Temperatur unterhalb des ersten vorbestimmten Temperaturwertes liegt, wobei der Schalttransistor (TR1) im Einschaltzustand die Erregung des Relais (RL1) und dadurch die Energiezufuhr zur Heizvorrichtung bewirkt.

26. Abfallauffangvorrichtung nach einem der Ansprüche 19 bis 25, weiterhin einen Zeitgeber (250) enthaltend, um die Heizvorrichtung (160) abzuschalten, nachdem sie für eine vorbestimmte Zeitdauer mit Energie versorgt worden ist.

27. Abfallauffangvorrichtung nach Anspruch 26, bei der der Zeitgeber (250) einen Zähler (IC7, IC8) aufweist, der zu Beginn des Betriebs der Heizvorrichtung mit einem Signal versorgt wird, um den Zähler (IC7, IC8) in den Zählzustand zu versetzen, eine zweite Torschaltung (IC6) vorhanden ist, der der Zähler nach einer vorbestimmten Zahl von Zählungen, die der vorbestimmten Zeitperiode entspricht, einen Ausgang zuführt, ein zweiter Schalttransistor (TR2) mit der zweiten Torschaltung (IC6) verbunden ist und ein Relais (RL1/1) vorhanden ist, das von dem zweiten Schalttransistor (TR2) so angesteuert wird, daß auf einen von dem Zähler (IC7, IC8) erzeugten Ausgang der zweite Schalttransistor das Relais (RL1/1) abschaltet, um damit die Heizvorrichtung (160) abzuschalten.

28. Abfallauffangvorrichtung nach einem der Ansprüche 19 bis 27, bei der der Gehäusetemperaturfühler (164) einen Balgen und einen Schalter (SW1) aufweist, derart, daß, wenn der Balgen sich ausdehnt, er den Schalter (SW1) öffnet, um die Heizvorrichtung (160) abzuschalten, wenn die Temperatur den zweiten vorbestimmten Wert übersteigt.

## Revendications

1. Dispositif permettant de détruire la flore bactérienne par stérilisation de résidus contaminés contenant la flore bactérienne, comprenant un boîtier (120), ledit boîtier possédant une entrée (122) permettant au matériau de résidus de passer dans ledit boîtier (120), et un moyen de chauffage (160) placé dans ledit boîtier pour chauffer le matériau de résidus de façon que toute la flore bactérienne qui se rassemble dans ou sur ledit boîtier puisse être stérilisée par la chaleur émanant dudit moyen de chauffage, afin de détruire ladite flore bactérienne, où ledit dispositif comporte en outre un moyen (130, IC2, IC3, IC4 A, B) servant à commander ledit moyen de chauffage de façon à maintenir la température régnant dans ledit boîtier au-dessus d'une première valeur prédéterminée, ledit moyen comportant un moyen capteur servant à mesurer (IC2) la température de l'eau dans le boîtier et à exciter le moyen de chauffage lorsque la température tombe au-dessous de la valeur prédéterminé, et un deuxième moyen capteur (164) servant à déterminer si la température régnant dans le boîtier dépasse une deuxième valeur prédéterminée et à faire que le moyen de chauffage soit désexcité si la température dépasse la deuxième valeur prédéterminée.

2. Dispositif selon la revendication 1, où le moyen de chauffage comprend un élément chauffant (160) et le boîtier comprend un cylindre (120) placé dans le bouchon d'eau d'un tuyau d'évacuation l'élément chauffant (160) s'étendant longitudinalement dans le cylindre (120) de façon que le matériau de résidus versé dans l'évier avec l'eau se rassemble dans le cylindre, si bien que l'eau est mise en ébullition par l'élément chauffant (160) et la chaleur est conduite dans le tuyau d'évacuation par l'intermédiaire de la vapeur et de la mise en ébullition de l'eau et du matériau de résidus afin de détruire les bactéries contenues dans l'eau ainsi que les bactéries qui se rassemblent sur l'intérieur du tuyau d'évacuation.

3. Dispositif selon la revendication 1 ou 2, où la première valeur prédéterminé de la température est 35°C et la deuxième valeur prédéterminée de la température est 120°C.

4. Dispositif selon l'une quelconque des revendications précédentes, où ledit dispositif comporte une sonde (170) de détection de bas niveau qui détecte lorsque le liquide contenu dans ledit boîtier tombe au-dessous d'un niveau prédéterminé et qui fait que le moyen de chauffage (160) est désexcité lorsque le niveau de liquide tombe au-dessous du niveau prédéterminé.

5. Dispositif selon l'une quelconque des revendications précédentes, où ledit deuxième moyen capteur comporte un soufflet (164).

6. Dispositif selon la revendication 4, où un moyen de commande électrique (220, 250, 260) est destiné à commander le fonctionnement dudit moyen de chauffage (160), ledit moyen de commande répondant au signal de sortie du moyen (IC2) capteur de température et de ladite sonde (170) de détection de niveau bas, ledit moyen de commande comportant un relais (RL1/3) qui est activé lorsque la température mesurée par le moyen capteur de température (IC2) est au-dessous de la valeur prédéterminée et lorsque le niveau d'eau mesuré par la sonde (170) de mesure de niveau bas est au-dessus du niveau prédéterminé, ledit relais, lorsqu'il est activé, fermant un moyen de commutation (RL1/1, ... 3) servant à autoriser l'excitation du moyen de chauffage (160).

7. Dispositif selon la revendication 7, où les signaux de sortie de la sonde (170) de mesure de niveau bas et du moyen capteur de température (IC2) sont appliqués à un moyen du type porte (IC4A, IC4B) qui rend conducteur un transistor de commutation (TR1) lorsque le niveau de liquide est au-dessus du niveau prédéterminé et la température est au-dessous de la température prédéterminée, ledit transistor de commutation (TR1), lorsqu'il est rendu conducteur, faisant que le relais (RL1/3) s'excite de manière à fermer ledit moyen de commutation (RL1/1, ... 3).

8. Dispositif selon l'une quelconque des revendications précédentes, où un moyen minuteur (250) est destiné à désexciter le moyen de chauffage (160) après que le moyen de chauffage (160) a été excité pendant un intervalle de temps prédéterminé.

9. Dispositif selon la revendication 8 prise en liaison avec la revendication 7 ou 8, où ledit moyen minuteur (250) comprend des moyens compteurs (IC7, IC8) qui reçoivent un signal au début de la marche du moyen de chauffage afin qu'ils soient positionnés et autorisés à compter, lesdits moyens compteurs appliquant un signal de sortie à un deuxième moyen du type porte (IC6C, IC4C, IC4D), après un nombre prédéterminé d'actions de comptage indicatif de l'intervalle de temps prédéterminé, afin de rendre non conducteur un deuxième transistor de commutation (TR2), et, ainsi, désactiver ledit relais (RL1/3) pour ouvrir le moyen de commutation (RL1/1, ... 3) et désexciter ledit moyen de chauffage (160).

10. Dispositif selon la revendication 5, où ledit capteur (164) de dépassement de température comporte un soufflet qui se dilate de manière à ouvrir un commutateur (SW1) pour désexciter ledit moyen de chauffage (160) lorsque la température dépasse le valeur prédéterminée.

11. Dispositif permettant de détruire la flore bactérienne par stérilisation de résidus contaminés contenant la flore bactérienne, ledit dispositif comprenant un boîtier (120), ledit boîtier possédant une entrée (122) permettant au matériau de résidus de passer dans ledit boîtier, et un moyen de chauffage (160) placé dans ledit boîtier (120) afin de chauffer le matériau de résidus de façon que toute la flore bactérienne qui se rassemble dans ou sur ledit boîtier puisse être stérilisée par la chaleur émanant dudit moyen de chauffage (160), où ledit dispositif comprend en outre un capteur (IC2) de température de liquide servant à mesurer le température du liquide à l'intérieur du boîtier (120), un capteur (164) de température du boîtier servant à mesurer la température du boîtier, une sonde (170) monté dans le boîtier afin de mesurer le niveau de liquide à l'intérieur du boîtier (120) pour effectuer des changements d'état si le niveau tombe au-dessous d'un niveau prédéterminé, et un moyen (250, 260, 130 IC2—4) de commande de température électriquement connecté au capteur de température de liquide (IC2), au capteur de température du boîtier, à la sonde et au moyen de chauffage, ledit moyen de commande de température (250, 260) étant conçu pour exciter le moyen de chauffage (160) pendant une durée prédéterminée si la température mesurée du liquide est en dessous d'une première valeur prédéterminée de température et si le niveau du liquide est au-dessus de son niveau prédéterminé; dans la measure où la température du boîtier reste inférieure à une deuxième valeur prédéterminé de température et où le niveau du liquide reste au-dessus de son niveau prédéterminé; si bien que le dispositif détruit de manière sûre la flore bactérienne associée aux résidus contaminés.

12. Dispositif selon la revendication 11, où le boîtier (120) comporte un moyen (126) permettant d'examiner l'intérieur du boîtier et où, de plus, le moyen de chauffage (160) comprend un élément chauffant et le boîtier comprend un cylindre placé dans un bouchon d'eau du tuyau d'évacuation, l'élément chauffant (160) s'étendant longitudinalement dans le cylindre de façon que le matériau de résidus rejeté dans l'évier avec l'eau se rassemble dans le cylindre, si bien que l'eau est mise en ébullition par l'élément chauffant et que la chaleur est conduite dans le tuyau d'évacuation par l'intermédiaire de la vapeur et de la mise en ébullition de l'eau et du matériau de résidus afin de détruire les bactéries contenues dans l'eau ainsi que les bactéries qui se sont rassemblées sur l'intérieur du tuyau d'évacuation.

13. Dispositif selon la revendication 11 ou 12, où le moyen de commande de température (250, 260, IC2—4, 132) comporte un relais (RL1) qui est activé lorsque la température mesurée par le capteur de température de liquide est en dessous de la première valeur prédéterminé de température et lorsque le niveau du liquide, mesuré par la sonde (170) est au-dessus du niveau prédéterminé, ledit relais (RL1) permettant, lorsqu'il est activé, l'excitation du moyen de chauffage.

14. Dispositif selon la revendication 13, où les signaux de sortie de la sonde (170) et du capteur de température de liquide (IC2) sont appliqués à un moyen du type porte (IC4) qui rend conducteur un transistor de commutation (TR1) lorsque le niveau de liquide est supérieur au niveau prédéterminé et la température est inférieure à la première valeur prédéterminée de température, ledit transistor de commutation faisant, lorsqu'il à été rendu conducteur, que le relais soit excité et activant donc le moyen de chauffage.

15. Dispositif selon la revendication 11, 12, 13 ou 14, comprenant en outre un moyen minuteur (250) servant à désexciter le moyen de chauffage (160) après que le moyen de chauffage (160) a été excité pendant la durée prédéterminée.

16. Dispositif selon la revendication 15, où ledit moyen minuteur comprend des moyens compteurs (IC7, IC8) qui reçoivent un signal au début de la marche du moyen de chauffage (160) de façon à être positionnés et à être autorisés à compter; un deuxième moyen du type porte (IC6), lesdits moyens compteurs (IC7, IC8) appliquant un signal de sortie audit deuxième moyen de type porte (IC6), après un nombre prédéterminé d'actions de comptage indicatif de l'intervalle de temps prédéterminé; un transistor de commutation (TR2) connecté au deuxième moyen du type porte et un relais (RL1/1) commandé par le transistor de commutation (TR2) de façon que, à le délivrance d'un signal de sortie par les moyens compteurs, le transistor de commutation désactive le relais afin de désexciter ledit moyen de chauffage.

17. Dispositif selon l'une quelconque des revendications 11 à 16, où le capteur (164) de température du boîtier comporte un soufflet et un commutateur (SW1), de sorte que, lorsque le soufflet se dilate, il ouvre le commutateur afin de désexciter ledit moyen de chauffage (160) lorsque la température dépasse la deuxième valeur prédéterminée de température.

18. Dispositif selon l'une quelconque des revendications 11 à 16, où ledit dispositif comporte un commutateur de repositionnement manuel (SW1) connecté au capteur (164) de température du boîtier afin d'empêcher que le moyen de chauffage (160) ne soit réactivé après avoir été désexcité suite au fait que la température du boîtier dépasse la deuxième valeur prédéterminée, avant que ledit commutateur (SW1), n'ait été repositionné manuellement.

19. Dispositif de piégeage de résidus permettant de détruire la flore bactérienne par stérilisation de résidus liquides contaminés contenant la flore bactérienne, ledit dispositif comprenant un boîtier allongé (120) comportant des moyens (122, 124) de raccordement à une tuyau d'évacuation, et un moyen de chauffage (160), où l'axe longitudinal du boîtier (120) est transversal à celui du tuyau d'évacuation de façon qu'il se forme au moins un bouchon d'eau et à ainsi faciliter le raccordement et l'entretien du dispositif, le moyen de chauffage (160) étant destiné à chauffer le matériau de résidus de façon que toute la flore bactérienne qui se rassemble dans ou sur ledit boîtier puisse être stérilisée par la chaleur émanant dudit moyen de chauffage, un moyen servant à commander (250, 260, IC2—4, 132) le moyen de chauffage (160) afin de maintenir la température des résidus liquides au-dessus d'une première valeur prédéterminée, ledit moyen de commande de température comprenant en outre un moyen (164) servant à mesurer la température dans le boîtier, ledit moyen (164) de mesure de la température étant placé dans une partie basse dudit boîtier, et une sonde (170) de mesure de niveau bas qui est montée dans une partie supérieure du boîtier afin de faire saillie dans ledit boîtier (120), la sonde (170) étant connectée audit moyen de chauffage (160) afin de détecter lorsque le liquide contenu dans le boîtier tombe au-dessous d'un niveau prédéterminé et de faire que le moyen de chauffage (160) soit désexcité lorsque le niveau de liquide tombe au-dessous de ce niveau prédéterminé; le moyen servant à commander (250, 260, IC2—4, 132) le moyen de chauffage répondant au moyen (164) de mesure de la température du boîtier en désexcitant le moyen de chauffage (160) lorsque le boîtier dépasse une deuxième valeur prédéterminée de température, si bien que le dispositif détruit de manière sûre la flore bactérienne associée aux résidus contaminés.

20. Dispositif de piégeage de résidus selon la revendication 19, comprenant en outre un commutateur manuellement actionnable (SW1) connecté au moyen (164) de mesure de la température du boîtier et au moyen servant à commander (250, 260, IC2—4, 132) le moyen de chauffage de façon à rendre obligatoire le repositionnement manuel du commutateur (SW1) lorsque la température du boîtier a dépassé la deuxième valeur prédéterminée de température.

21. Dispositif de piégeage de résidus selon la revendication 19 ou 20, où le boîtier comprend en outre une ouverture d'inspection à l'une de ses extrémités, afin de faciliter l'entretien et l'inspection du dispositif.

22. Dispositif de piégeage de résidus selon la revendication 20, où le boîtier allongé (126) comprend un cylindre (120) destiné à être placé dans le bouchon d'eau du tuyau d'évacuation, où le moyen de chauffage (160) comprend un élément chauffant (160) s'étendant longitudinalement dans le cylindre de façon que les résidus liquides jetés dans un évier associé au tuyau d'évacuation se rassemblent dans le cylindre (120) et soient mis en ébullition par l'élément chauffant au moyen de la chaleur conduite dans le tuyau d'évacuation par l'intermédiaire de la vapeur et de la mise en ébullition des résidus liquides afin de détruire les bactéries contenues dans les résidus liquides ainsi que les bactéries qui se rassemblent sur l'intérieur du tuyau d'évacuation.

23. Dispositif de piégeage de résidus selon la revendication 22, où le moyen de commande du moyen de chauffage (250, 260, IC2) comporte un capteur de température de liquide (IC2) servant à mesurer la température du liquide à l'intérieur du boîtier.

24. Dispositif de piégeage de résidus selon la revendication 23, où le moyen servant à commander (250, 260, IC2—4, 132) le moyen de chauffage comporte un relais (RL1) qui est activé lorsque la température mesurée par le capteur de température de liquide (IC2) est au-dessous d'une première valeur prédéterminée et lorsque le niveau de liquide mesuré par la sonde (170) de mesure de niveau bas est au-dessus du niveau prédéterminé; ledit relais (RL1) permettant, lorsqu'il est activé, l'excitation du moyen de chauffage (160).

25. Dispositif de piégeage de résidus selon la revendication 24, où le moyen servant à commander (250, 260, IC2—4, 132) le moyen de chauffage (160) comporte un moyen du type porte (IC4) et un transistor de commutation (TR1), le moyen du type porte (IC4) étant connecté aux sorties de la sonde (170) de mesure de niveau bas et du capteur de température de liquide (IC2), de façon à rendre conducteur le transistor de commutation (TR1) lorsque le niveau de liquide est au-dessus du niveau prédéterminé et que la température est au-dessous de la première valeur prédéterminée de température, le transistor de commutation (TR1) faisant, lorsqu'il est rendu conducteur, que le relais (RL1) soit excité et active donc le moyen de chauffage.

26. Dispositif de piégeage de résidus selon l'une quelconque des revendications 19 à 25, comprenant en outre un moyen minuteur (250) servant à désexciter le moyen de chauffage (160) après que celui-ci a été excité pendant une durée prédéterminée.

27. Dispositif de piégeage de résidus selon la revendication 26, où le moyen minuteur (250) comprend des moyens compteurs (IC7, IC8) qui reçoivent un signal au début de la marche du moyen de chauffage afin d'être repositionnés et de commencer à compter, un deuxième moyen du type porte (IC6), les moyens compteurs appliquant un signal de sortie au deuxième moyen du type porte (IC6) après un nombre prédéterminé d'actions de comptage indicatif de la durée prédéterminée; un deuxième transistor de commutation (TR2) connecté au deuxième moyen du type porte (IC6) et un relais (RL1/1) commandé par le deuxième transistor de commutation (TR2) de sorte que, à la délivrance d'un signal de sortie par les moyens compteurs (IC7, IC8), le deuxième transistor de commutation désactive le relais (RL1/1)/ afin de désexciter le moyen de chauffage (160).

28. Dispositif de piégeage de résidus selon l'une quelconque des revendications 19 à 27, où le capteur (164) de température du boîtier comporte un soufflet et un commutateur (SW1) de sorte que, lorsque le soufflet se dilate, il ouvre le commutateur (SW1) afin de désexciter le moyen de chauffage (160) lorsque la température dépasse la deuxième valeur prédéterminée.

FIG. 1.

FIG. 2.